# EUROPEAN PATENT SPECIFICATION

(11) **EP 1 611 920 B1**
(45) Date of publication and mention of the grant of the patent: **13.05.2009**
(21) Application number: 04717796.9
(22) Date of filing: 05.03.2004
(51) Int. Cl.: A61M 29/02

(54) **STENT**
STENT
STENT

(30) Priority: 10.03.2003 JP 2003064224
(43) Date of publication of application: 04.01.2006
(73) Proprietor: KANEKA CORPORATION, Osaka-shi, Osaka 530-8288 (JP)
(72) Inventor: FUKAYA, Kohei, c/o Kaneka Corporation, Osaka 566-0072 (JP); NAKANO, Ryohji, c/o Kaneka Corporation, Osaka 566-0072 (JP); TANI, Nobutaka, c/o Kaneka Corporation, Osaka 566-0072 (JP)
(74) Representative: Gille Hrabal Struck Neidlein Prop Roos
(86) International application number: PCT/JP2004/002885
(87) International publication number: WO 2004/080521

(56) References cited:
- EP-A1- 0 565 796
- WO-A-91/12779
- WO-A2-20/04060460
- JP-A- 05 293 176
- JP-A- 05 502 179
- JP-A- 11 501 526
- JP-A- 2001 502 192
- US-A- 5 593 417
- US-B1- 6 197 013

## Description

### Technical Field

The present invention relates to medical stents for preventing or treating stenosis of body passages, in particular, blood vessels.

### Background Art

Angioplasty (percutaneous transluminal angioplasty (PTA) and percutaneous transluminal coronary angioplasty (PTCA)) is a lowly invasive, widespread technique for treating stenosed or occluded blood vessels by inflating small balloons in blood vessels. However, according to this technique, repeated stenosis (restenosis) occurs at a high incidence. As the technique for reducing the incidence of restenosis, atherectomy, laser treatment, radiotherapy, and the like have been attempted. Another technique that has gained popularity in recent years is stent placement.

A stent is a medical device for curing various disorders inflicted by stenosis or occlusion of blood vessels or other body lumina. The stent is placed at the stenosed or occluded site to expand the affected site and to maintain the lumen size, and is usually composed of a metal or a polymer. The stent is typically inserted to a blood vessel using a balloon catheter and is expanded to provide mechanical support to the intravascular lumen by coming into contact with the affected area of the arterial wall. Although the stent placement has been proven to significantly reduce the incidence of restenosis compared to techniques that use only balloons, restenosis still occurs at a high incidence. For example, restenosis has been reported in about 20% to 30% of cases of stent placement in coronary arteries.

Some of the restenosis cases are induced by biological vascular injuries and others by vascular injuries caused by stent placement. It is generally established that typical angiostenosis and restenosis caused by vascular injuries occur due to proliferation of intimal smooth muscle cells. First, proliferation of smooth muscle cells is initiated after vascular injury, and then the smooth muscle cells migrate to the intima. The smooth muscle cells in the intima then proliferate, accompanied with substrate deposition, thereby causing intimal thickening. The T-cells, macrophages, and the like are also considered to migrate to the intima.

In order to reduce the incidence of restenosis, studies have been made from various aspects. One approach is to reduce vascular injuries by increasing the flexibility of the stent itself, improving the design of the stent, or the like.

Japanese Patent No. 2591573 discloses a balloon catheter including a balloon and a plurality of atheroma cutters (metal blades attached to a dilatable member with a polyurethane adhesive) attached to the outer surface of the balloon to reduce the stress applied to blood vessels during the dilation procedure. It is possible that a procedure of implanting a stent after dilation using this balloon catheter is effective.

Another approach is to decrease the restenosis rate by coating the stent with a drug that prevents restenosis, as disclosed in PCT Japanese Translation Patent Publication No. 5-502179. As the drug that inhibits occlusion, many drugs have been investigated including anticoagulants, antiplatelets, anticonvulsants, antibacterials, antitumor agents, antimicrobials, antiinflammatory agents, antimetabolics, and immunosuppressants. With respect to immunosuppressants, an attempt for reducing restenosis by coating a stent with an immunosuppressant, such as cyclosporin, tacrolimus (FK-506), sirolimus (rapamycin), mycophenolate mofetil, or an analogue of any one of these has been made. For example, PCT Japanese Translation Patent Publication No. 5-502179 discloses a stent coated with a known immunosuppressant, sirolimus (rapamycin). PCT Japanese Translation Patent Publication No. 9-503488 discloses a stent coated with an antitumor agent, Taxol (paclitaxel). Moreover, WO 02/065947 and EP 1254674 each disclose a stent coated with tacrolimus (FK-506).

US 6,197,013 B1 discloses a stent comprising a stent body being a substantially tubular body extendable in the outward radial direction of the substantially tubular body and a structure for cutting body tissues, whereby the structure is disposed on an outer surface of the stent body. However, US 6,197,013 B1 does not teach that the structure of the stent is elongated In the axial direction of the substantially tubular body.

WO 2004/060460 A2 relates to a medical device, such as medical balloons, catheters, etc., having one or more cutting elements. Thereby the cutting devices are fully adhered to the medical device.

### Disclosure of Invention

An object of the present invention is to provide a stent that can further reduce the incidence of repeated stenosis (restenosis) that has been experienced with conventional stents or drug-coated stents.

In order to achieve the object, a structure for cutting body tissues is disposed on the outer surface of the stent, and the stent is expanded and placed while cutting the body tissues, such as blood vessels. In this manner, the stress and the unrestricted damage applied to the body tissues such as blood vessels can be reduced, and restenosis can be suppressed. The stent of the present invention includes a substantially tubular stent body radially expandable in an outward direction and a structure for cutting the body tissues, the structure being disposed on the outer surface of the stent body.

Embodiments of stents of the present invention will now be described.

The stent of the present invention includes a substantially tubular stent body radially expandable in an outward direction and a structure for cutting body tissues, disposed on the outer surface of the stent body.
Fig. 1 is a development elevation of an example of a stent body 1 according to the present invention.
Fig. 2 is a schematic diagram showing an embodiment of the stent of the invention. Onto the outer surface of the expanded stent body 1, a structure 2 for cutting body tissues is disposed. The structure 2 is wire-shaped, has a circular cross-section, and extends in the axial direction.
Fig. 3 is a schematic cross-sectional view of the stent body 1 provided with a plurality of the wire-shaped structures 2 having a circular cross-section and extending in the axial direction disposed on the outer surface of the stent body 1. Fig. 3 is viewed in the axial direction of the stent body.

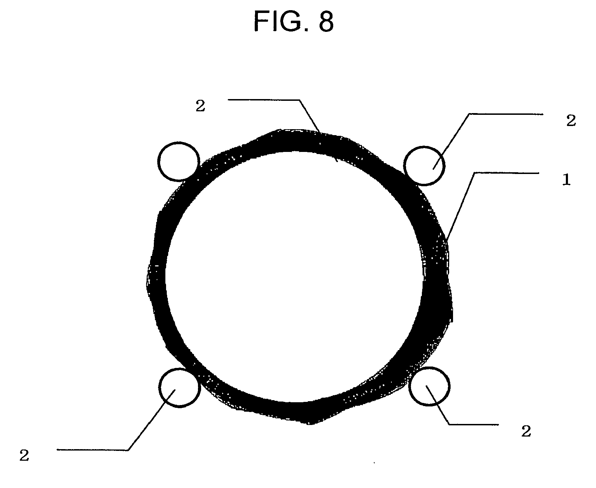

Fig. 8 is a schematic cross-sectional view of the stent body provided with four structures 2 described above as viewed in the axial direction of the stent body. In this embodiment, examples including three or four structures 2 are provided; however the number of the structures 2 may be larger. The structures protrude in the outward radial direction of the stent and thus cut the body tissues once pressed against the body tissues by the stent expansion. In this embodiment, the material and the shape of each structure are not particularly limited as long as the structure is suitable for achieving the intended functions. The structure is elongated in the axial direction and a circular or elliptic cross-section is preferred since such a structure advantageously renders flexibility to the stent. In this embodiment, the maximum height of the structure in the outward radial direction of the stent body should not be excessively small since the cutting performance becomes insufficient but should not be excessively large since the flexibility of the stent is impaired and significant damage is inflicted upon the body. The maximum height is preferably 0.10 mm to 0.60 mm and more preferably 0.20 mm to 0.40 mm.

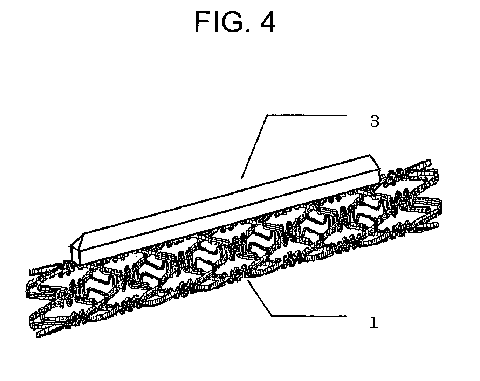

Fig. 4 is a schematic diagram showing another embodiment of the stent of the present invention. Onto the outer surface of the expanded stent body 1, a metal blade 3 for cutting body tissues is disposed. The metal blade 3 is directed in the outward radial direction of the stent body.

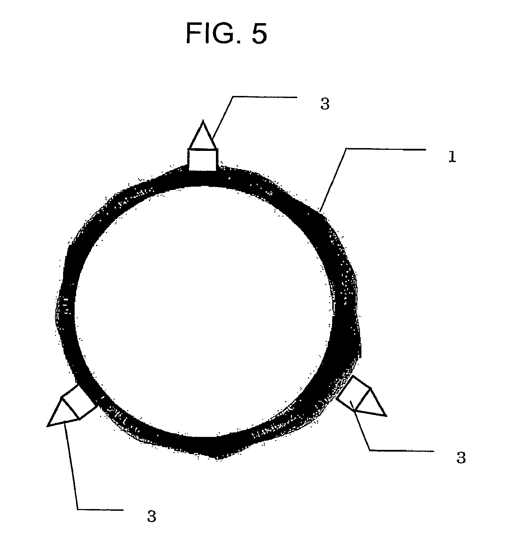

Fig. 5 is a schematic cross-sectional view of the stent body provided with a plurality of structures 3, which are metal blades, as viewed in the axial direction of the stent body.

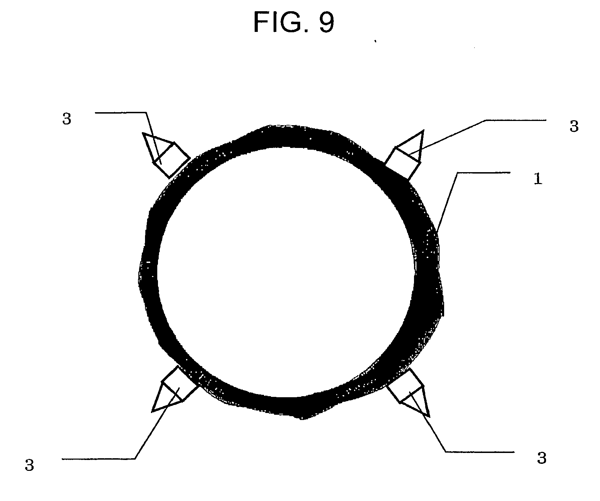

Fig. 9 is a schematic cross-sectional view of the stent body provided with four structures 3 as viewed in the axial direction of the stent body. In this embodiment, examples having three or four structures 3 are provided; however, the number of the structures 3 may be larger. In this embodiment, the structures, i.e., the metal blades, have edges directed in the outward radial direction of the stent body. When the stent is expanded, the structures are pressed against and thereby cut the body tissues. In this embodiment, the properties and the shape of each blade are not particularly limited. Preferably, the edge of the blade is prepared by two or more stages of abrasion so that the blade has a high body-cutting property. The angle of the first abrasion is preferably 25° to 56°. The angle of the second abrasion is preferably 30° to 70°. The maximum height of the structure, i.e., the metal blade, in the outward radial direction of the stent body should not be excessively small since the cutting performance becomes insufficient but should not be excessively large since the flexibility of the stent is impaired and significant damage is inflicted upon the body. The maximum height is preferably 0.10 mm to 0.60 mm and more preferably 0.10 mm to 0.30 mm.

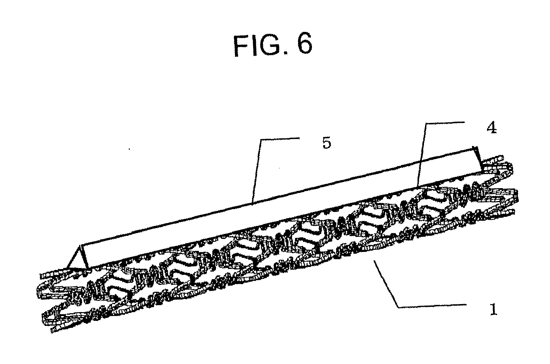

Fig. 6 is a schematic diagram of yet another embodiment of the stent of the invention. In this embodiment, a structure 4 for cutting body tissues is disposed on the outer surface of the expanded stent body 1. The structure 4 has an edge 5 directed to the outward radial direction of the stent body and is composed of a polymeric material.

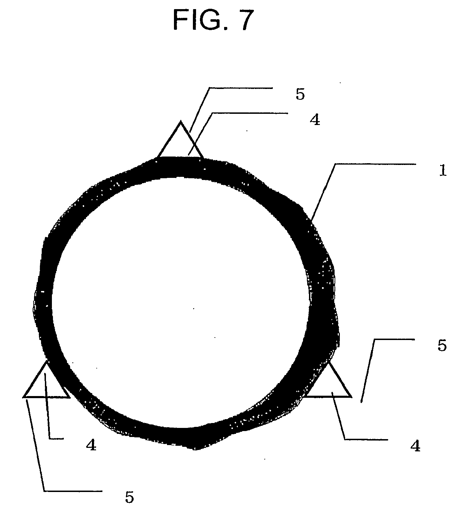

Fig. 7 is a schematic cross-sectional view of a stent having a plurality of the structures 4 having the edges 5 directed in the outward radial direction of the stent body and being composed of a polymeric material, as viewed in the axial direction of the stent body.

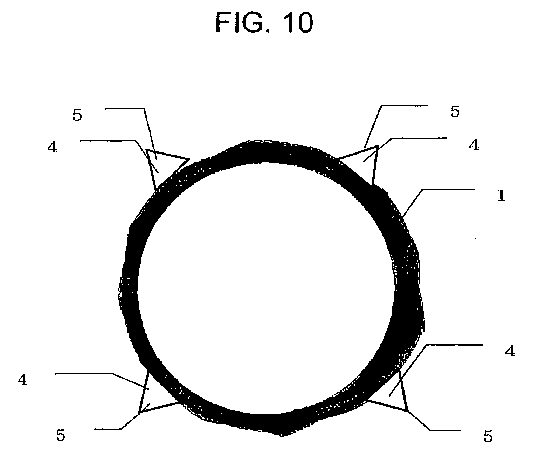

Fig. 10 is a schematic cross-sectional view of a stent having four structures 4 as viewed in the axial direction of the stent body. In this embodiment, examples having three or four structures 4 are given; however, the number of the structures 4 may be larger. In this embodiment, when the stent is expanded, the structures having the edges 5 directed in the outward radial direction are pressed against and thereby cut the body tissues. In this embodiment, the material and the shape of the structure having the edge directed in the outward radial direction are not particularly limited. In order to yield a body-cutting property, the angle of the edge is preferably rather small, e.g., 120° or less. The angle is more preferably 90° or less since the size of the structure increases with the angle of the edge. In this embodiment, the maximum height of the structure in the outward radial direction, the structure having the edge directed in the outward radial direction of the stent body, should not be excessively small since the cutting performance becomes insufficient but should not be excessively large since the flexibility of the stent is impaired and significant damage is inflicted upon the body. The maximum height is preferably 0.10 mm to 0.60 mm and more preferably 0.15 mm to 0.35 mm.

In this invention, the methods of disposing and fixing the structure for cutting the body tissues on the stent are not particularly limited. The structure is preferably disposed parallel to the stent axis since, in most cases, the structure can be advantageously folded and mounted onto the balloon catheter for dilation. Various methods for fixing can be adopted depending on the materials of the stent and the structure directed in the outward radial direction. When both the stent and the structure are composed of metal, the stent and the structure are preferably welded from the standpoint of strength. When the structure is composed of a polymeric material, although bonding with an adhesive is possible, it is preferable to process the polymeric material into a shape attachable to the stent. The position of fixing the structure is also not particularly limited. In order to comply with the bending and expanding of the stent, the structure is preferably fixed onto the stent body at as few positions as possible.

In this invention, the structure for cutting the body tissues may be composed of metal, a polymeric material, or a composite material of metal and a polymer. When the structure is entirely or at least partially composed of a polymeric material, a drug may be contained in the polymeric material.

The polymeric material usable in the present invention is preferably one that does not easily induce deposition of platelets, that does not irritate tissues, and that allows elution of the drug. A polymeric material having high biocompatibility is particularly preferable. Examples of the preferred synthetic polymeric materials include a blend or block copolymer of a polyether-type polyurethane and dimethylsilicon, polyurethanes such as segmented polyurethane, polyacrylamide, polyethylene oxide, and polycarbonates such as polyethylene carbonate and polypropylene carbonate. Examples of preferred natural polymeric materials include fibrins, gelatins, and collagens. These polymeric materials may be used alone or in combination as required.

The biodegradable polymeric material usable in the present invention may be any but preferably one that is enzymatically or non-enzymatically decomposed in vivo, yields no toxic decomposition product, and has ability of releasing the drug. For example, a suitable one selected from polylactic acid, polyglycolic acid, a polylactic acid-polyglycolic acid copolymer, collagen, gelatin, chitin, chitosan, hyaluronic acid, polyamino acids such as poly-L-glutamic acid and poly-L-lysine, starch, poly-ε-caprolactone, polyethylene succinate, poly-β-hydroxyalkanoate, and the like may be used. These polymeric materials may be used alone or in combination as required.

The polymeric material for the structure for cutting the body tissues is not particularly limited as long as the polymeric material has a particular hardness for cutting purposes. Since the structure is preferably absorbed in the body after the placement, a biodegradable polymeric material is preferable. Polylactic acid and a polylactic acid-polyglycolic acid copolymer are particularly preferred. When the stent is composed of a polymeric material or coated with a polymeric material, the structure is preferably composed of a polymeric material the same as or weldable to the polymeric material that constitutes or coats the stent from the standpoint of processing. More preferably, a polylactic acid-based polymeric material, which is biodegradable, is used for both the stent body and the structure. When the stent is coated with a polymeric material or a drug, the polymeric material or the drug must have a certain level of flexibility to comply with the displacement caused by the stent expansion; on the other hand, the structure is sometimes required to have a certain degree of hardness and preferable biodegradable properties. In such a case, suitable polymeric materials may be respectively used. For example, a polyurethane and a polylactic acid-glycol acid copolymer are preferable for coating the stent. The ratio of the lactic acid component (PL) to the glycol acid component (PG) of the polylactic acid-glycol acid copolymer, i.e., the ratio PL/GA, is preferably set to a value that facilitates the formation of the coating layer, e.g., 75/25 to 50/50. Polylactic acid and the polylactic acid-glycol copolymer are suitable for use in the structure. If accelerated biodegradation of the structure is desired, the ratio of the GA may be increased to the maximum within the above-described preferable range. In some cases, the ratio PL/GA of 50/50 can be advantageously used.

The stent of the invention may include a stent body coated with a polymeric material. The polymeric material in the form of liquid or a solution in an adequate solvent such as water, a buffer, acetic acid, hydrochloric acid, methanol, ethanol, acetone, acetonitrile, methylene chloride, chloroform, tetrahydrofuran, or the like may be spread onto the stent, followed by solvent removal to obtain a stent coated with the polymeric material. As the method for spreading the polymeric material solution onto the stent, for example, a dipping method in which the stent is dipped in the solution or a spraying method using a sprayer may be employed.

The stent of the invention may include a stent body coated with a drug. As for the application method, the drug may be directly applied onto the stent. It is more preferable to apply onto the stent body a polymeric material containing or impregnated with a drug since the drug can be more easily retained. The polymeric material is preferably a biocompatible or biodegradable polymeric material. The drug may be applied onto the stent by spreading the solution of the drug onto the stent, followed by solvent removal. The polymeric material in the form liquid or a solution in an adequate solvent, such as water, a buffer, acetic acid, hydrochloric acid, methanol, ethanol, acetone, acetonitrile, methylene chloride, chloroform, tetrahydrofuran, or the like may be spread onto the stent, and the solvent may then be removed to obtain a stent coated with the polymeric material. As the method for spreading the polymeric material solution onto the stent, for example, a dipping method in which the stent is dipped in the solution or a spraying method using a sprayer may be employed. The drug may be contained in the solvent so that the mixture of the polymeric material and the drug can be applied onto the stent. Alternatively, the stent coated with the polymeric material may be immersed in the drug solution so that the drug is impregnated in the polymeric material.

Similarly, the structure may be coated with the drug. Only the structure may be coated with the drug, or both the stent and the structure may be coated with the drug. In the latter case, the type of the drug and the amount of the drug may be the same or different between the structure and the stent body. However, since the structure is positioned at the incision site, the drug preferably has an anti-inflammatory effect or an anti-inflammatory immunosuppressive effect such as one exhibited by tacrolimus. In some cases, the drug content is preferably higher for the structure than the drug content in the stent coating since the drug can intensively and effectively work at the incision site. When the structure is entirely or at least partly composed of a polymeric material, the drug may be contained in the polymeric material. The polymeric material is preferably biodegradable since it can be absorbed in the body after cutting and drug releasing. The polymeric material is more preferably polylactic acid since hardness necessary for cutting and excellent drug applicability, drug-retaining ability, and bioabsorbability can be yielded.

The drug preferably has restenosis preventive effects and is preferably an immunosuppressant or an antiinflammatory agent. Examples of the immunosuppressant include cyclosporin, tacrolimus (FK-506), mycophenolate mofetil, sirolimus (rapamycin), and analogues thereof (everolimus, ABT-578, CCI-779, AP23573, etc.). Examples of the antiinflammatory agent include adrenocortical steroids and non-steroids such as dexamethasone, hydroxycortisone, cortisone, desoxycorticosterone, fludrocortisone, betamethasone, prednisolone, prednisone, methylprednisolone, paramethasone, triamcinolone, flumetasone, fluocinolone, fluocinonide, fluprednisolone, halcinonide, flurandrenolide, meprednisone, medrysone, cortisol, 6α-methylprednisolone, triamcinolone, betamethasone, salicylic acid derivatives, diclofenac, naproxen, sulindac, and indomethacin. Tacrolimus (FK506) is a compound of CAS No. 104987-11-3 disclosed in Japanese Unexamined Patent Application Publication No. 61-148181, for example. Tacrolimus (FK506) forms a complex with an intracellular FK-506-binding protein (FKBP), thereby primarily inhibiting production of cytokines, such as differentiators/growth stimulators, IL-2 and INF-γ, from the T-cells. It is a well-known fact that tacrolimus can be used as a drug for preventing or curing immunological rejection in organ transplants or autoimmune diseases. Furthermore, tacrolimus (FK506) has been confirmed to have antiproliferative effects on human vascular cells (Paul J. Mohacsi MD, et al., The Journal of Heart and Lung Transplantation, May 1997, vol. 16, No. 5, 484-491); hence, use of tacrolimus is also preferable. Dexamethasone is an adrenocortical steroid of CAS No. 50-02-2 having antiinflammatory and antiallergic effects and affects metabolism of sugars, proteins, fats, and the like. Its applications to chromic rheumatoid arthritis, bronchial asthma, atopic dermatitis, and the like are well known. Indomethacin is a nonsteroidal compound of CAS No. 58-86-1 having antiinflammatory effects. Its applications to chromic rheumatoid arthritis, antiinflammatory agents, analgesics, and antipyretics are known. Thus, use of indomethacin is also preferable. The drug is not limited to these and various drugs effective for restenosis can be used. Examples thereof include antiproliferation agents; etoposide, actinomycin D, cisplatin, vincristine, irinotecan hydrochloride, and antiplatelet agents; sarpogrelate, trapidil, aspirin, dipyridamole, ticlopidine, abciximab, a vascular endothelium growth factor (VEGF), a fibroblast growth factor (FGF), a nucleic acid medicine NFKB, and ACE inhibitor cilazapril.

In order to prevent the structure for cutting body tissues from cutting unaffected blood vessels during the delivery of the inventive stent toward the target site, a separate protection mechanism may be provided. Various protection mechanisms are usable for the inventive stent. For example, a flexible protective plate may be disposed on the side surface of the structure. A conceivable configuration here is a protective plate having an outermost end in the radial direction located at a higher position than the outermost end of the structure in the radial direction. The protective mechanism exhibits the protection function during the delivery, but is pressed open to allow cutting during the stent expansion. Alternatively, for example, a protective mechanism may be mounted to the catheter itself for delivering the stent.

### Brief Description of the Drawings

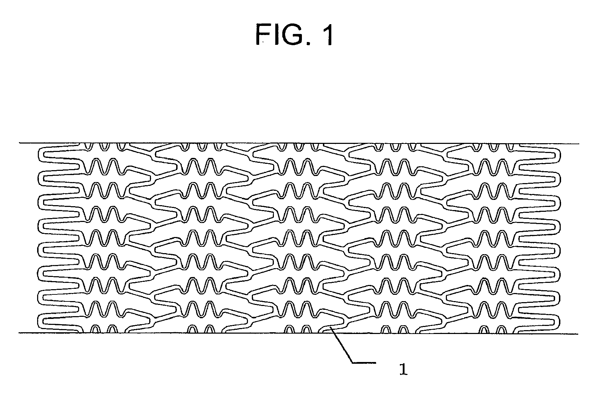

Fig. 1 is a schematic view (development elevation) of a stent 1.

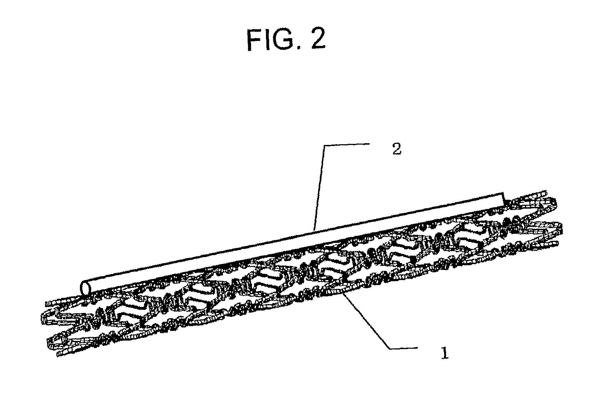

Fig. 2 is a schematic view showing an example of the stent of the present invention.

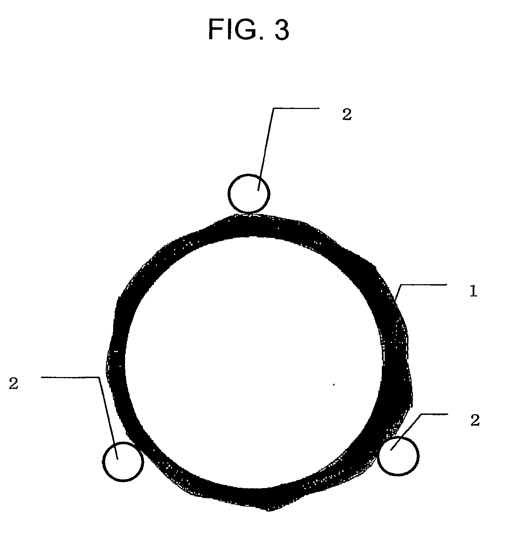

Fig. 3 is a schematic cross-sectional view showing an example of the stent of the present invention.
Fig. 4 is a schematic view showing an example of the stent of the present invention.
Fig. 5 is a schematic cross-sectional view showing an example of the stent of the present invention.
Fig. 6 is a schematic view showing an example of the stent of the present invention.
Fig. 7 is a schematic cross-sectional view showing an , example of the stent of the present invention.
Fig. 8 is a schematic cross-sectional view showing an example of the stent of the present invention.
Fig. 9 is a schematic cross-sectional view showing an example of the stent of the present invention.
Fig. 10 is a schematic cross-sectional view showing an example of the stent of the present invention.

### Reference Numerals

- 1: stent body
- 2: structure for cutting body tissues, the structure having the shape of a wire and a circular cross-section
- 3: metal blade
- 4: structure for cutting body tissues, the structure having an edge
- 5: edge directed in the outward radial direction of the stent body

### Best Mode for Carrying Out the Invention

### (EXAMPLE 1)

Onto the outer surface of a stainless-steel stent body shown in the development elevation of Fig. 1, four stainless-steel wires having a diameter of 0.4 mm were mounted as structures for cutting body tissues so as to extend parallel to the axial direction of the stent body, as shown in Fig. 8. The wires were equally spaced and each wire was welded onto the outer surface at a center point. A stent having a nominal expansion diameter of 3.5 mm and a length of 13 mm in the axial direction was prepared thereby.

### (EXAMPLE 2)

The stent prepared in EXAMPLE 1 was spray-coated with a polyurethane resin and dipped in an ethanol solution of tacrolimus to prepare a stent in which the stent body and the structures for cutting body tissues were coated with a polymeric material and tacrolimus impregnated in the polymeric material. The tacrolimus content in the coating was controlled to 200 µg (0.20 mg) per stent.

### (EXAMPLE 3)

Onto the outer surface of a stainless-steel stent body shown in the development elevation of Fig. 1, four stainless-steel blades having a height of 0.3 mm, an angle of first abrasion of 45°, and an angle of second abrasion of 56° were mounted as structures for cutting body tissues so as to extend parallel to the axial direction of the stent body, as shown in Fig. 9. The blades were equally spaced and each blade was welded onto the outer surface at two points near the two ends. A stent having a nominal expansion diameter of 3.5 mm and a length of 13 mm in the axial direction was prepared thereby.

### (EXAMPLE 4)

The stent prepared in EXAMPLE 3 was spray-coated with a polyurethane resin and dipped in an ethanol solution of tacrolimus to prepare a stent in which the stent body and the structures for cutting body tissues were coated with a polymeric material and tacrolimus impregnated in the polymeric material. The tacrolimus content in the coating was controlled to 200 µg (0.20 mg) per stent.

### (EXAMPLE 5)

Onto the outer surface of a stainless-steel stent body shown in the development elevation of Fig. 1, mounted were four structures for cutting body tissues, each structure being made of polylactic acid, having a height of 0.3 mm with an edge directed at an angle of 60° in the outward radial direction of the stent body, and being an elongated structure having a triangular cross-section, as shown in Fig. 10. The structures extended parallel to the axial direction of the stent body and were attached to the outer surface of the stent body at equal intervals each at two points at the two ends of the structure. A stent having a nominal expansion diameter of 3.5 mm and a length of 13 mm in the axial direction was prepared thereby.

### (EXAMPLE 6)

The stent prepared in EXAMPLE 5 was spray-coated with a polyurethane resin and dipped in an ethanol solution of tacrolimus to prepare a stent in which the stent body and the structures for cutting body tissues were coated with a polymeric material and tacrolimus impregnated in the polymeric material. The tacrolimus content in the coating was controlled to 200 µg (0.20 mg) per stent.

### (EXAMPLE 7)

Onto the outer surface of a stainless-steel stent body shown in the development elevation of Fig. 1, mounted were four structures for cutting body tissues, each structure being made of polylactic acid containing 10 percent by weight of tacrolimus, having a height of 0.3 mm with an edge directed at an angle of 60° in the outward radial direction of the stent body, and being an elongated structure having a triangular cross-section, as shown in Fig. 10. The structures extended parallel to the axial direction of the stent body and were attached onto the outer surface of the stent body at equal intervals at two points at the two ends of each structure. A stent having a nominal expansion diameter of 3.5 mm and a length of 13 mm in the axial direction was prepared thereby. The tacrolimus content was 320 µg (0.32 mg) per stent.

### (EXAMPLE 8)

A stainless-steel stent body shown in the development elevation of Fig. 1 was spray-coated with a polyurethane resin and dipped in an ethanol solution of tacrolimus to coat the stent body with 150 µg of tacrolimus. Onto the resulting stent body, mounted were four structures for cutting body tissues, each structure being made of polylactic acid containing 10 percent by weight of tacrolimus, having a height of 0.3 mm with an edge directed at an angle of 60° in the outward radial direction of the stent body, and being an elongated structure having a triangular cross-section, as shown in Fig. 10. The structures extended parallel to the axial direction of the stent body and were attached to the outer surface of the stent body at equal intervals at two points at the two ends of each structure. A stent having a nominal expansion diameter of 3.5 mm and a length of 13 mm in the axial direction was prepared thereby. The tacrolimus content was 470 µg (0.47 mg) per stent.

### (EXAMPLE 9)

A stainless-steel stent body shown in the development elevation of Fig. 1 was spray-coated with a polyurethane resin and dipped in an ethanol solution of tacrolimus to coat the stent body with 150 µg of tacrolimus. Onto the resulting stent body, mounted were four structures for cutting body tissues, each structure being made of polylactic acid containing 6 percent by weight of dexamethasone, having a height of 0.3 mm with an edge directed at an angle in the outward radial direction of the stent body of 60°, and being an elongated structure having a triangular cross-section, as shown in Fig. 10. The structures extended parallel to the axial direction of the stent body and were attached to the outer surface of the stent body at equal intervals at two points at the two ends of each structure. A stent having a nominal expansion diameter of 3.5 mm and a length of 13 mm in the axial direction was prepared thereby. The tacrolimus content was 150 µg (0.15 mg) per stent, and the dexamethasone content was 200 µg (0.20 mg) per stent.

### (EXAMPLE 10)

Onto the outer surface of a stainless-steel stent body shown in the development elevation of Fig. 1, mounted were four structures for cutting body tissues, each structure being made of a polylactic acid/glycol acid copolymer having a PL/GA ratio of 50/50 and containing 10 percent by weight of tacrolimus, having a height of 0.3 mm with an edge directed at an angle of 60° in the outward radial direction of the stent body, and being an elongated structure having a triangular cross-section, as shown in Fig. 10. The structures extended parallel to the axial direction of the stent body and were attached to the outer surface of the stent body at equal intervals at two points at the two ends of each structure. A stent having a nominal expansion diameter of 3.5 mm and a length of 13 mm in the axial direction was prepared thereby. The tacrolimus content was 200 µg (0.20 mg) per stent.

### (EXAMPLE 11)

A stainless-steel stent body shown in the development elevation of Fig. 1 was spray-coated with a polylactic acid/glycol acid copolymer having a PL/GA ratio of 75/25 and containing 20 percent by weight of tacrolimus. The coating amount was controlled such that the tacrolimus content was 200 µg (0.20 mg). As shown in Fig. 10, onto the outer surface of the stent body, mounted were four structures for cutting body tissues, each structure being made of a polylactic acid/glycol acid copolymer having a PL/GA ratio of 50/50 and containing 10 percent by weight of tacrolimus, having a height of 0.3 mm with an edge directed at an angle of 60° in the outward radial direction of the stent body, and being an elongated structure having a triangular cross-section. The structures extended parallel to the axial direction of the stent body and were attached onto the outer surface of the stent body at equal intervals at two points at the two ends of each structure. A stent having a nominal expansion diameter of 3.5 mm and a length of 13 mm in the axial direction was prepared thereby. The tacrolimus content was 400 µg (0.40 mg) per stent.

### (EXAMPLE 12)

A stainless-steel stent body shown in the development elevation of Fig. 1, was spray-coated with a polylactic acid/glycol acid copolymer having a PL/GA ratio of 50/50 and containing 20 percent by weight of tacrolimus. The coating amount was controlled such that the tacrolimus content was 200 µg (0.20 mg). As shown in Fig. 10, onto the outer surface of the stent body, mounted were four structures for cutting body tissues, each structure being made of a polylactic acid/glycol acid copolymer having a PL/GA ratio of 50/50 and containing 10 percent by weight of tacrolimus, having a height of 0.3 mm with an edge directed at an angle of 60° in the outward radial direction of the stent body, and being an elongated structure having a triangular cross-section. The structures extended parallel to the axial direction of the stent body and were attached to the outer surface of the stent body at equal intervals at two points at the two ends of each structure. A stent having a nominal expansion diameter of 3.5 mm and a length of 13 mm in the axial direction was prepared thereby. The tacrolimus content was 400 µg (0.40 mg) per stent.

### (COMPARATIVE EXAMPLE)

A stainless-steel stent shown in the development elevation of Fig. 1 having a nominal expansion diameter of 3.5 mm and a length of 13 mm in the axial direction.

### (COMPARATIVE EXPERIMENT 1)

Each rabbit (New Zealand white, male, 13-14 weeks old) was intubated at the left femoral artery, and a Fogarty arterial embolectomy catheter 3F (length: 80 cm) was inserted through the left femoral artery to the aorta abdominalis. The balloon was inflated at a site 7 cm from the branch of the femoral artery (caliber of the vessel: about 3 mm) and slightly slid to inflict injuries in the inner wall of the vessel. The intubation site was sutured, and the blood flow was regenerated. Subsequently, the rabbit was given a diet of 10% cholesterol for 1 month to induce angiostenosis in the aorta abdominalis. Each of the stents of EXAMPLES 1 to 9 and COMPARATIVE EXAMPLE at the angiostenosis site was expanded to 1.2 times the original caliber of the blood vessel and left at the site. On the day of the placement experiment and the days immediately before and after the placement experiment, 40 mg of aspirin was forcibly fed. Starting from two days after the stent placement, a feed of 10% cholesterol containing 40 mg of aspirin was given until the removal. One month after the placement, three section samples were respectively taken from a proximal site (upstream of the artery, near the heart), the center, and a distal site (downstream of the artery relative to the proximal site) of the stent. The samples were stained with hematoxylin eosin (HE) and Elastica-van Gieson (EVG) to prepare observation samples. As the evaluation items, the vascular lumen area (LA) and the area within the internal elastic lamina (IELA) of each stent cross-section were determined. Furthermore, the vascular lumen occlusion rate (%) was calculated by (1 - (LA/IELA)) × 100, from the observed vascular lumen area (LA) and the area within the internal elastic lamina (IELA). The evaluation results after one month of the stent placement according to COMPARATIVE EXPERIMENT 1 are shown in Table 1. The figures show the averages of the respective groups. In EXAMPLES 1 to 9 of the present invention, favorable results, i.e., smaller vascular lumen occlusion rates, were observed at the respective observation sites compared with those of COMPARATIVE EXAMPLE. The samples equipped with structures for cutting body tissues, the structures coated with or containing drugs, showed better results.

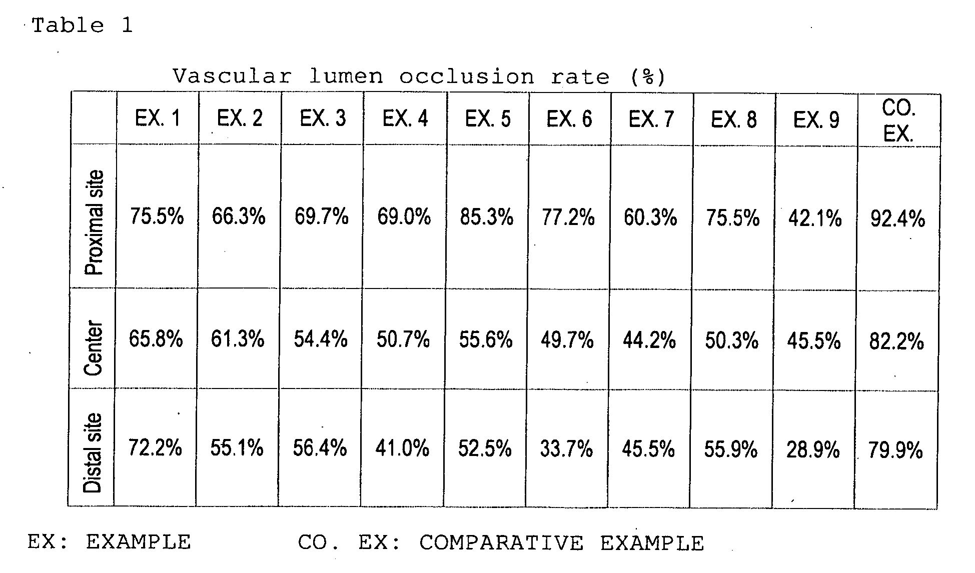

### (COMPARATIVE EXPERIMENT 2)

Each rabbit (New Zealand white, male, 13-14 weeks old) was intubated at the left femoral artery, and a stent coated with copper by sputtering was delivered to a site 7 cm from the branch of the femoral artery (caliber of the vessel: about 3 mm), expanded to 1.3 times the caliber of the blood vessel, and left at the site. The intubation site was sutured, and the blood flow was regenerated. Subsequently, the rabbit was given a diet of 10% cholesterol for two weeks to induce stent restenosis in the aorta abdominalis. Each of the stents of EXAMPLES 1 to 9 and COMPARATIVE EXAMPLE delivered to the restenosis site was expanded to 1.2 times the original caliber of the blood vessel and left at the site. On the day of the placement experiment and the days immediately before and after the placement experiment, 40 mg of aspirin was forcibly fed. Starting from two days after the stent placement, a feed of 10% cholesterol containing 40 mg of aspirin was given until the removal. One month after the placement, three section samples were respectively taken from a proximal site (upstream of the artery, near the heart), the center, and a distal site (downstream of the artery relative to the proximal site) of the stent. The vascular lumen occlusion rate (%) of each sample was determined as in COMPARATIVE EXPERIMENT 1. Table 2 shows the evaluation results after one month of the stent placement in COMPARATIVE EXPERIMENT 2. The figures show the averages of the respective group. In EXAMPLES 1 to 9 of the present invention, favorable results, i.e., smaller vascular lumen occlusion rates, were observed at the respective observation sites compared with those of COMPARATIVE EXAMPLE. The samples equipped with structures for cutting body tissues, the structures coated with or containing drugs, showed better results.

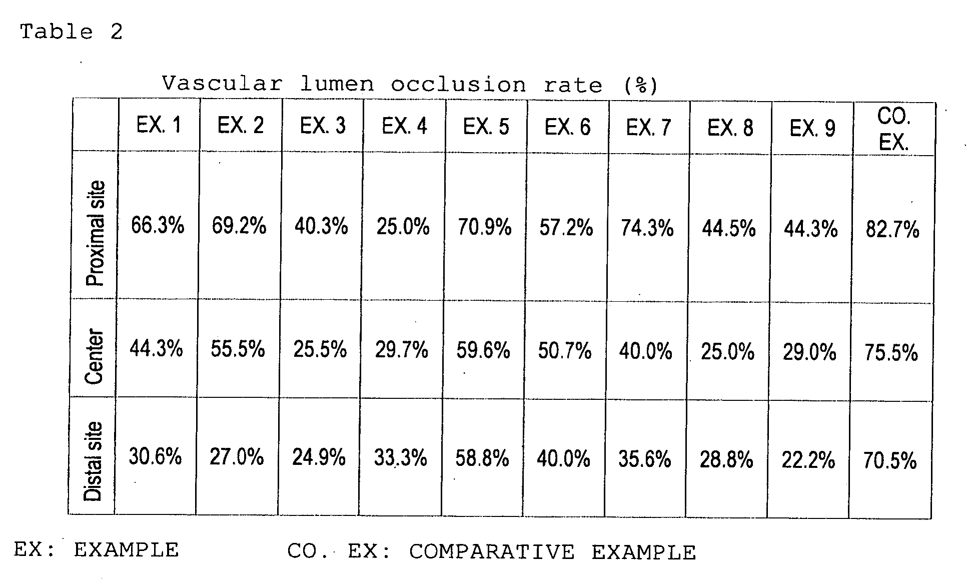

### (COMPARATIVE EXPERIMENT 3)

The stents of EXAMPLE 8 and COMPARATIVE EXAMPLE were respectively placed at the left anterior descending branch and the left circumflex branch of the left coronary artery of a miniature pig (male, 10 month old, 35 kg) and expanded to 1.2 times the original caliber of the respective blood vessels. Subsequently, 325 mg of aspirin and 250 mg of ticlopidine were fed daily. One month after the placement, three section samples were respectively taken from a proximal site (upstream of the artery, near the heart), the center, and a distal site (downstream of the artery relative to the proximal site) of the stent. The vascular lumen occlusion rate (%) of each sample was determined as in COMPARATIVE EXPERIMENT 1. Table 3 shows the evaluation results of COMPARATIVE EXPERIMENT 2 after one month of the stent placement. The figures show the averages of the respective group. In EXAMPLES 8, 10, 11, and 12 of the present invention, favorable results, i.e., smaller vascular lumen occlusion rates, were observed at the respective observation sites compared with those of COMPARATIVE EXAMPLE.

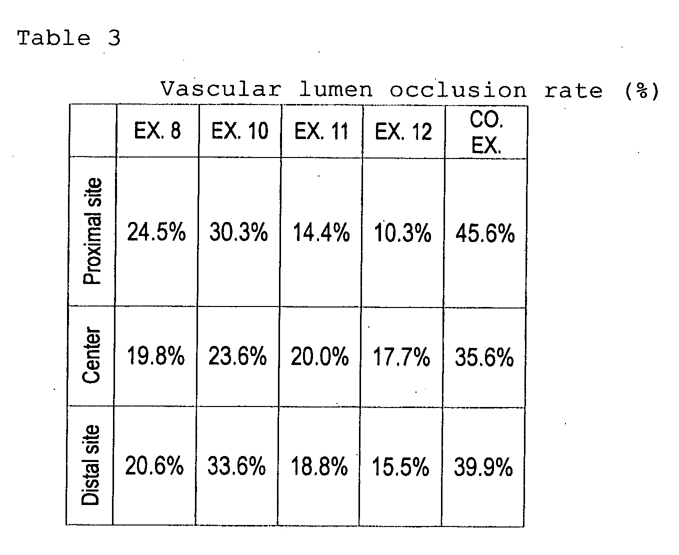

### (COMPARATIVE EXPERIMENT 4)

Each rabbit (New Zealand white, male, 13-14 weeks old) was intubated at the left femoral artery, and each of the stents of EXAMPLES 5, 7, 8, and 9 and COMPARATIVE EXAMPLE was delivered to a site 7 cm from the branch of the femoral artery (caliber of the vessel: about 3 mm), expanded to 1.2 times the caliber of the blood vessel, and left at the site. On the day of the placement experiment and the days immediately before and after the placement experiment, 40 mg of aspirin was forcibly administered. Starting from two days after the stent placement, a feed containing 40 mg of aspirin was given until the removal. Four months after the placement, a section sample was taken from the center of each stent and was stained as in COMPARATIVE EXPERIMENT 1 to observe the state of the buried stent. In EXAMPLES 5, 7, 8, and 9 of the present invention having structures composed of biodegradable polylactic acid for cutting body tissues, most of the structures were absorbed in the body after four months, and it was confirmed that no significant adverse effect was inflicted upon the peripheral tissues as with the stent of COMPARATIVE EXAMPLE.

### Industrial Applicability

An excellent stent that reduces the incidence of the stenosis, restenosis, and in-stent restenosis that occur with stents is provided.

## Claims

1. A stent comprising a stent body being a substantially tubular body expandable in the outward radial direction of the substantially tubular body and a structure for cutting body tissues, the structure disposed on an outer surface of the stent body, **characterized in that** the structure is elongated in the axial direction of the substantially tubular body and wherein the structure for cutting the body tissues is fixed onto the stent body at one or two points.

2. The stent according to claim 1, wherein the structure for cutting the body tissues is fixed onto the stent body at a center point.

3. The stent according to claim 1, wherein the structure for cutting the body tissues is fixed onto the stent body at the two ends of the structure.

4. The stent according to any of claims 1 to 3, wherein the structure for cutting the body tissue is fixed onto the stent body In such a way as to keep the structure elongated in the axial direction of the substantially tubular body before and after expansion.

5. The stent according to any of claims 1 to 4, comprising a plurality of the structures.

6. The stent according to any of claims 1 to 5, wherein the structure has a circular or elliptic cross-section.

7. The stent according to any one of claims 1 to 6, wherein the structure has an edge directed in the outward radial direction of the stent body when the stent body is expanded.

8. The stent according to claim 7, wherein the angle of the edge is 120° or less.

9. The stent according to claim 7 or 8, wherein the structure has a triangular cross-section.

10. The stent according to any one of claims 1 to 9, wherein the material of the structure is a metal.

11. The stent according to any one of claims 1 to 9, wherein the material of the structure is a polymeric material.

12. The stent according to any one of claims 1 to 9, wherein the material of the structure is a composite material of a metal and a polymer.

13. The stent according to claim 12, wherein the polymeric material is applied onto the metal material.

14. The stent according to any one of claims 11 to 13, wherein the polymeric material is a biodegradable polymeric material.

15. The stent according to claim 14, wherein the material of the structure is polylactic acid.

16. The stent according to any one of claims 7, 8 or 10, wherein the structure is a metal blade having a cutting edge directed in the outward radial direction of the stent body when the stent body is expanded.

17. The stent according to any one of claims 1 to 16. wherein the maximum height of the structure in the outward radial direction of the stent body is 0.1 to 0.6 mm when the stent body is expanded.

18. The stent according to any one of claims 1 to 17, wherein the stent body is coated with a drug.

19. The stent according to claim 18, wherein the stent body is coated with a polymeric material, and the drug is contained or impregnated in the polymeric material.

20. The stent according to any one of claims 1 to 19wherein the structure is coated with a drug.

21. The stent according to any one of claims 13 to 20, wherein at least part of the structure comprises a polymeric material, and the polymeric material contains a drug.

22. The stent according to any one of claims 18 to 21, wherein the drug has an effect of preventing restenosis.

23. The stent according to any one of claims 18 to 22, wherein the drug is tacrolimus (FK-506), its analogue, or dexamethasone.

## Patentansprüche

1. Ein Stent, umfassend einen Stentkörper, welcher ein im Wesentlichen röhrenförmiger Körper ist, welcher in die radiale Außenrichtung des im Wesentlichen röhrenförmigen Körpers expandierbar ist und eine Struktur aufweist, um Körpergewebe abzuschneiden, wobei die Struktur auf der äußeren Oberfläche des Stentkörpers vorgesehen ist, **dadurch gekennzeichnet, dass** die Struktur in die axiale Richtung des im Wesentlichen röhrenförmigen Körpers gestreckt ist und worin die Struktur zum Abschneiden der Körpergewebe auf dem Stentkörper an einem oder mehreren Punkten fixiert ist.

2. Der Stent nach Anspruch 1 , worin die Struktur zum Schneiden der Körpergewebe auf dem Stentkörper an einem zentralen Punkt fixiert ist.

3. Der Stent nach Anspruch 1 , worin die Struktur zum Schneiden der Körpergewebe auf dem Stentkörper an den zwei Enden der Struktur fixiert ist.

4. Der Stent nach einem der Ansprüche 1 bis 3, worin die Struktur zum Abschneiden des Körpergewebes so auf dem Stentkörper fixiert ist, dass die Struktur vor und nach der Expansion in die axiale Richtung des im Wesentlichen röhrenförmigen Körpers gestreckt bleibt.

5. Der Stent nach einem der Ansprüche 1 bis 4, welcher eine Mehrzahl an den Strukturen umfasst.

6. Der Stent nach einem der Ansprüche 1 bis 5, worin die Struktur einen zirkularen oder elliptischen Querschnitt aufweist.

7. Der Stent nach einem der Ansprüche 1 bis 6, worin die Struktur eine Kante aufweist, welche in die radiale Auswärtsrichtung des Stentkörpers gerichtet ist, wenn der Stentkörper expandiert wird.

8. Der Stent nach Anspruch 7, worin der Winkel der Kante 120 ° oder weniger beträgt.

9. Der Stent nach Anspruch 7 oder 8, worin die Struktur einen dreieckigen Querschnitt aufweist.

10. Der Stent nach einem der Ansprüche 1 bis 9, worin das Material der Struktur ein Metall ist.

11. Der Stent nach einem der Ansprüche 1 bis 9, worin das Material der Struktur ein polymeres Material ist.

12. Der Stent nach einem der Ansprüche 1 bis 9, worin das Material der Struktur ein Verbundmaterial eines Metalles und eines Polymers ist.

13. Der Stent nach Anspruch 12, worin das polymere Material auf dem Metallmaterial aufgebracht ist.

14. Der Stent nach einem der Ansprüche 11 bis 13, worin das polymere Material ein bioabbaubares polymeres Material ist.

15. Der Stent nach Anspruch 14, worin das Material der Struktur Polymilchsäure ist.

16. Der Stent nach einem der Ansprüche 7, 8 oder 10, worin die Struktur ein Metallmesser mit einer Schneidekante ist, welche in die radiale äußere Richtung des Stentkörpers gerichtet ist, wenn der Stentkörper expandiert wird.

17. Der Stent nach einem der Ansprüche 1 bis 16, worin die maximale Höhe der Struktur in die radiale Auswärtsrichtung des Stentkörpers 0,1 bis 0,6 mm ist, wenn der Stentkörper expandiert wird.

18. Der Stent nach einem der Ansprüche 1 bis 17, worin der Stentkörper mit einem Arzneimittel beschichtet ist.

19. Der Stent nach Anspruch 18, worin der Stentkörper mit einem polymeren Material beschichtet ist und das Arzneimittel in dem polymeren Material enthalten oder imprägniert ist.

20. Der Stent nach einem der Ansprüche 1 bis 19, worin die Struktur mit einem Arzneimittel beschichtet ist.

21. Der Stent nach einem der Ansprüche 13 bis 20, worin mindestens ein Teil der Struktur ein polymeres Material umfasst und das polymere Material ein Arzneimittel umfasst.

22. Der Stent nach einem der Ansprüche 18 bis 21, worin das Arzneimittel die Wirkung aufweist, die Restenosis zu verhindern.

23. Der Stent nach einem der Ansprüche 18 bis 22, worin das Arzneimittel Tacrolismus (FK-506), sein Analoges oder Dexamethason ist.

## Revendications

1. Endoprothèse vasculaire comprenant un corps d'endoprothèse vasculaire, à savoir un corps essentiellement tubulaire apte à se déployer dans la direction radiale vers l'extérieur du corps essentiellement tubulaire, et une structure pour la découpe de tissus corporels, la structure étant disposée sur une surface externe du corps de l'endoprothèse vasculaire, **caractérisée en ce que** la structure est allongée dans la direction axiale du corps essentiellement tubulaire, la structure pour la découpe de tissus corporels étant fixée sur le corps de l'endoprothèse vasculaire à un ou deux endroits.

2. Endoprothèse vasculaire selon la revendication 1, dans laquelle la structure pour la découpe des tissus corporels est fixée sur le corps de l'endoprothèse vasculaire à un point central.

3. Endoprothèse vasculaire selon la revendication 1, dans laquelle la structure pour la découpe des tissus corporels est fixée sur le corps de l'endoprothèse vasculaire aux deux extrémités de la structure.

4. Endoprothèse vasculaire selon l'une quelconque des revendications 1 à 3, dans laquelle la structure pour la découpe du tissu corporel est fixée sur le corps de l'endoprothèse vasculaire de façon à maintenir la structure allongée dans la direction axiale du corps essentiellement tubulaire avant et après son déploiement.

5. Endoprothèse vasculaire selon l'une quelconque des revendications 1 à 4, comprenant plusieurs structures.

6. Endoprothèse vasculaire selon l'une quelconque des revendications 1 à 5, dans laquelle la structure possède une section transversale circulaire ou elliptique.

7. Endoprothèse vasculaire selon l'une quelconque des revendications 1 à 6, dans laquelle la structure possède un bord orienté dans la direction radiale vers l'extérieur du corps de l'endoprothèse vasculaire lorsque le corps de l'endoprothèse vasculaire est déployé.

8. Endoprothèse vasculaire selon la revendication 7, dans laquelle l'angle formé par le bord s'élève à 120° ou moins.

9. Endoprothèse vasculaire selon la revendication 7 ou 8, dans laquelle la structure possède une section transversale triangulaire.

10. Endoprothèse vasculaire selon l'une quelconque des revendications 1 à 9, dans laquelle la matière de la structure est un métal.

11. Endoprothèse vasculaire selon l'une quelconque des revendications 1 à 9, dans laquelle la matière de la structure est une matière polymère.

12. Endoprothèse vasculaire selon l'une quelconque des revendications 1 à 9, dans laquelle la matière de la structure est un matériau composite d'un métal et d'un polymère.

13. Endoprothèse vasculaire selon la revendication 12, dans laquelle la matière polymère est appliquée sur la matière métallique.

14. Endoprothèse vasculaire selon l'une quelconque des revendications 11 à 13, dans laquelle la matière polymère est une matière polymère biodégradable.

15. Endoprothèse vasculaire selon la revendication 14, dans laquelle la matière de la structure est l'acide polylactique.

16. Endoprothèse vasculaire selon l'une quelconque des revendications 7, 8 ou 10, dans laquelle la structure est une lame métallique possédant un bord tranchant orienté dans la direction radiale vers l'extérieur du corps de l'endoprothèse vasculaire lorsque le corps de l'endoprothèse vasculaire est déployé.

17. Endoprothèse vasculaire selon l'une quelconque des revendications 1 à 16, dans laquelle la hauteur maximale de la structure dans la direction radiale vers l'extérieur du corps de l'endoprothèse vasculaire s'élève de 0,1 à 0,6 mm lorsque le corps de l'endoprothèse vasculaire est déployé.

18. Endoprothèse vasculaire selon l'une quelconque des revendications 1 à 17, dans laquelle le corps de l'endoprothèse vasculaire est enduit d'un médicament.

19. Endoprothèse vasculaire selon la revendication 18, dans laquelle le corps de l'endoprothèse vasculaire est enduit d'une matière polymère, et le médicament est contenu ou imprégné dans la matière polymère.

20. Endoprothèse vasculaire selon l'une quelconque des revendications 1 à 19, dans laquelle la structure est enduite d'un médicament.

21. Endoprothèse vasculaire selon l'une quelconque des revendications 13 à 20, dans laquelle au moins une partie de la structure comprend une matière polymère et la matière polymère contient un médicament.

22. Endoprothèse vasculaire selon l'une quelconque des revendications 18 à 21, dans laquelle le médicament a pour effet de prévenir une resténose.

23. Endoprothèse vasculaire selon l'une quelconque des revendications 18 à 22, dans laquelle le médicament est le tacrolimus (FK-506), son analogue ou la dexaméthasone.
